# EUROPÄISCHE PATENTSCHRIFT

(11) **EP 1 390 518 B1**
(45) Veröffentlichungstag und Bekanntmachung des Hinweises auf die Patenterteilung: **06.10.2004**
(21) Anmeldenummer: 02740284.1
(22) Anmeldetag: 23.04.2002
(51) Int. Cl.: C12N 15/87, A61K 48/00, A61K 38/00, A61K 47/00, G01N 33/50

(54) **PUFFERLÖSSUNG FÜR DIE ELEKTROPORATION UND VERFAHREN UMFASSEND DIE VERWENDUNG DERSELBEN**
BUFFER SOLUTION FOR ELECTROPORATION AND A METHOD COMPRISING THE USE OF THE SAME
SOLUTION TAMPON DESTINEE A L'ELECTROPORATION ET PROCEDE UTILISANT CETTE SOLUTION

(30) Priorität: 23.04.2001 DE 10120000
(43) Veröffentlichungstag der Anmeldung: 25.02.2004
(73) Patentinhaber: Amaxa GmbH, 50829 Köln (DE)
(72) Erfinder: ROTHMANN-Cosic Kirsten, 10117 Berlin (DE); WESSENDORF, Heike, D-50676 Köln (DE); HELFRICH, Juliana, 08371 Glauchau (DE); THIEL, Corinna, 50823 Köln (DE); RIEMEN, Gudula, 40764 Langenfeld (DE); BROSTERHUS, Helmut, 57399 Kirchundern (DE); MÜLLER-HARTMANN, Herbert, 50823 Köln (DE); WEIGEL, Meike, 51067 Köln (DE); LORBACH, Elke, 51103 Köln (DE); NIX, Michael, 50937 Köln (DE); SIEBENKOTTEN, Gregor, 50226 Frechen-Königdorf (DE)
(86) Internationale Anmeldenummer: PCT/DE2002/001483
(87) Internationale Veröffentlichungsnummer: WO 2002/086134

(56) Entgegenhaltungen:
- EP-A- 0 414 551
- WO-A-02/00871
- WO-A-95/06129
- WO-A-99/13719
- US-B1- 6 184 254
- YE X ET AL: "ISOLATION OF LACTOPEROXIDASE, LACTOFERRIN, ALPHA-LACTALBUMIN, BETA-LACTOGLOBULIN B AND BETA-LACTOGLOBULIN A FROM BOVINE RENNET WHEY USING ION EXCHANGE CHROMATOGRAPHY" INTERNATIONAL JOURNAL OF BIOCHEMISTRY AND CELL BIOLOGY, EXETER, GB, Bd. 32, Nr. 11, November 2000 (2000-11), Seiten 1143-1150, XP001064940 ISSN: 1357-2725
- TISHCHENKO G A ET AL: "Effect of salt concentration gradient on separation of different types of specific immunoglobulins by ion-exchange chromatography on DEAE cellulose" JOURNAL OF CHROMATOGRAPHY B: BIOMEDICAL SCIENCES & APPLICATIONS, ELSEVIER SCIENCE PUBLISHERS, NL, Bd. 706, Nr. 1, 27. Februar 1998 (1998-02-27), Seiten 157-166, XP004110842 ISSN: 1570-0232
- BOLZACCHINI E ET AL: "PURIFICATION OF PHLEUM PRATENSE POLLEN EXTRACT BY IMMUNOAFFINITY CHROMATOGRAPHY AND HIGH-PERFORMANCE ION-EXCHANGE CHROMATOGRAPHY" JOURNAL OF CHROMATOGRAPHY, ELSEVIER PUBLISHING COMPANY, AMSTERDAM, NL, Bd. 548, Nr. 1/2, 1991, Seiten 229-234, XP001007426
- YAN CHANG-NING ET AL: "High-voltage and high-salt buffer facilitates electroporation of human aortic smooth-muscle cells." BIOTECHNIQUES, Bd. 24, Nr. 4, April 1998 (1998-04), Seiten 590-592, XP001153825 ISSN: 0736-6205 in der Anmeldung erwähnt
- NEUMANN E ET AL: "GENE TRANSFER INTO MOUSE LYOMA CELLS BY ELECTROPORATION IN HIGH ELECTRIC FIELDS" EMBO JOURNAL, IRL PRESS, EYNSHAM, GB, Bd. 1, Nr. 7, 1. Juli 1982 (1982-07-01), Seiten 841-845, XP002038197 ISSN: 0261-4189 in der Anmeldung erwähnt

## Beschreibung

Die Erfindung betrifft eine Pufferlösung zum Suspendieren tierischer oder menschlicher Zellen und Lösen biologisch aktiver Molekülen zum Zwecke des Einbringens dieser biologisch aktiven Moleküle in die Zellen mittels elektrischen Stroms sowie ein Verfahren zum Einbringen von biologisch aktiven Molekülen in tierische oder menschliche Zellen mittels elektrischen Stroms, umfassend die Verwendung der Pufferlösung.

### Hintergrund

Das Einbringen biologisch aktiver Moleküle, wie beispielsweise DNA, RNA oder Proteine, in lebende Zellen stellt ein wichtiges Instrument zur Untersuchung biologischer Funktionen dieser Moleküle dar. Eine bevorzugte Methode zum Einbringen von Fremdmolekülen in die Zellen ist dabei die Elektroporation, welche im Gegensatz zu anderen Methoden nur geringe anhaltende Veränderungen der biologischen Struktur und Funktionen der Zielzelle durch die Transferreagenzien bewirkt. Bei der Elektroporation werden die Fremdmoleküle aus einer wässrigen Lösung durch einen kurzzeitigen Stromfluss in die Zellen eingebracht, wobei durch die Wirkung der kurzen elektrischen Impulse die Zellmembran für die Fremdmoleküle durchlässig gemacht wird. Durch die kurzzeitig entstehenden "Poren" in der Zellmembran gelangen die biologisch aktiven Moleküle zunächst in das Zytoplasma, in dem sie ggf. bereits ihre zu untersuchende Funktion ausüben können. Im Falle des Einbringens von DNA in eukaryontische Zellen, muss diese aber in den Zellkern gelangen, um eine Expression der genetischen Information zu ermöglichen. Dies kann bei teilungsaktiven Zellen während der Zellteilung erfolgen, wobei die DNA nach der vorübergehenden Auflösung der Kernhülle passiv in den Zellkern gelangt. Bei Untersuchungen an ruhenden oder schwach teilungsaktiven Zellen, beispielsweise primären tierischen Zellen, gelangt die DNA aber nicht auf diese Weise in den Zellkern, so dass entsprechende Verfahren hier nicht anwendbar oder zumindest sehr langwierig sind. Insbesondere beim Einbringen von DNA in tierische Zellen, der sogenannten Transfektion, entstehen ferner häufig aufgrund der Labilität der Zellen besondere Probleme bei der Elektroporation, da die Überlebensrate der Zellen als wichtiger Parameter die Effizienz der Transfektion beeinflusst.

### Stand der Technik

Zur Aufnahme der tierischen Zellen und der DNA-Moleküle während der Elektroporation wurden in der Vergangenheit häufig Zellkulturmedien (Anderson et al. (1991), J. Biochem. Biophys. Meth. 22, 207) oder mit Phosphat oder HEPES gepufferte Salzlösungen (Potter et al. (1984), Proc. Natl. Acad. Sci. USA 81, 7161; Fromm et al. (1985), Nature 319, 791) eingesetzt. Es wurden aber auch nicht oder schwach gepufferte Mannitol- oder Saccharose-Lösungen bei der Elektroporation von tierischen Zellen verwendet (Shimizu et al. (1986), Med. Immunol. 11, 105; Riggs et al. (1986), Proc. Natl. Acad. Sci. USA 83, 5602). Solche nicht oder schwach gepufferte Lösungen haben aber den Nachteil, dass ihre Verwendung zu einer erhöhten Zellmortalität und deutlich verringerten Transfektionseffizienz führt (Yan et al. (1998), BioTechniques 24, 590).

Yan et al. (1998) verwendeten daher eine Pufferlösung für die Elektroporation bestehend aus 100 mM HEPES, 137 mM NaCl, 4 mM Na₂HPO₄ und 6 mM Dextrose. In diesem Puffer konnten zwar glatte Muskelzellen aus humaner Aorta erfolgreich transfiziert werden, jedoch betrug hier die Transfektionseffizienz lediglich 15 % bei einer Überlebensrate von nur 10 bis 20 %. Ferner ist der von Yan et al. verwendete Puffer lediglich für Spannungspulse bis 500 V optimiert, so dass keine Hinweise darauf gegeben werden, ob dieser Puffer auch bei höheren Spannungspulsen, wie sie z.B. für die direkte Transfektion in den Zellkern benötigt werden, verwendet werden kann. In jedem Fall sind aber die in diesem Puffer erzielten Transfektionseffizienzen zu gering, um höheren Ansprüchen gerecht zu werden.

Vielfach wurden Puffer mit geringer lonenstärke und damit geringer Leitfähigkeit verwendet, um Zellschäden aufgrund hoher Ströme zu vermeiden, was bei der Verwendung von Pufferlösungen mit hoher Leitfähigkeit, insbesondere beim Anlegen von längeren Hochspannungspulsen, beobachtet wurde.

Friedrich et al., 1998 (Bioelectrochem. and Bioenerg. 47, 103) konnten zeigen, dass der Stromfluss bei der Elektroporation durch Elektrolyse des Wassers zu einer Änderung des pH-Wertes in der Nähe der Elektroden führt. Diese Änderung des pH-Wertes bedingt die Freisetzung von cytotoxischen Al³⁺-lonen aus den Aluminiumelektroden der Küvetten und damit eine erhöhte Zellmortalität. Die Autoren schlagen hier zur Erhöhung der Transfektionseffizienz eine Verkürzung der Pulsdauer vor. Hinweise auf eine Änderung des verwendeten Puffers werden nicht gegeben.

Auch divalente Kationen, wie beispielsweise Mg²⁺, werden dem Elektroporationspuffer häufig zugegeben. Magnesiumionen erleichtern die Bindung von DNA an die Oberfläche der Zellen und bewirken dadurch eine erhöhte Transfektionsrate. Allerdings scheint dies nur für Mg²⁺-Konzentrationen bis zu 10 mM zu gelten, da bei höheren Konzentrationen negative Effekte, wie beispielsweise Verringerung der Elektrophorese durch Neutralisation der Ladungen der DNA-Moleküle oder Erhitzung des Puffers durch Erhöhung der Leitfähigkeit, überwiegen (Xie and Tsong (1993), Biophys. J. 65, 1684); Neumann et al. (1982), EMBO J. 1, 841; Klenchin et al. (1991), Biophys. J. 60, 804).

Femer wurde vorgeschlagen, den Puffer hinsichtlich seiner Zusammensetzung an die intrazellulären Verhältnisse anzupassen, um die Überlebensrate der Zellen zu steigern. Es sollte daher ein Puffer mit dem Zytoplasma entsprechender, hoher Kalium- und geringer Natriumkonzentration verwendet werden, damit ein Zusammenbruch des intrazellulären Na⁺/K⁺-Verhältnisses durch Stoffaustausch über die während der Elektroporation entstehenden Poren in der Zellmembran verhindert wird (van den Hoff et al. (1995), Methods in Mol. Biol. 48, Chapter 15, 185-197). Allerdings wurde hier eine Verringerung der Transfektionseffizienz bei Pulsen mit höherer Feldstärke ab 1300 V/cm festgestellt.

Alle bisher beschriebenen Pufferlösungen haben aber den Nachteil, dass die bei ihrer Verwendung erzielten Transfektionseffizienzen relativ gering sind und/oder die Puffer für die Anwendung bei der Elektroporation von ruhenden oder schwach teilungsaktiven Zellen nicht geeignet sind.

### Beschreibung der Erfindung

Der Erfindung liegt daher die Aufgabe zugrunde, eine Pufferlösung für die Elektroporation zur Verfügung zu stellen, welche höhere Transfektionseffizienzen bei geringer Zellmortalitätsrate ermöglicht, sowie ein entsprechendes Verfahren zu entwickeln.

Die Aufgabe wird dadurch gelöst, dass die erfindungsgemäße Pufferlösung bei einer Änderung des pH-Wertes von pH 7 nach pH 8 und einer Temperatur von 25 °C eine Pufferkapazität von mindestens 20 mmol * I⁻¹ * pH⁻¹ und eine Ionenstärke von zumindest 200 mmol * I⁻¹ aufweist, und bei der die Konzentration an Kalium-Ionen (K⁺) 2 bis 6 mmol * I⁻¹ K⁺ und die Konzentration an Natrium-Ionen (Na⁺) 100 bis 150 mmol * I⁻¹ Na⁺ beträgt. Mit einer solchen Pufferlösung ist das Einbringen biologisch aktiver Moleküle in tierische oder menschliche Zellen mittels Elektroporation mit Transfektionseffizienzen von bis zu 93% bei gleichzeitig geringer Zellmortalität möglich. Es können in diesem Puffer verschiedenen Zelltypen, insbesondere auch ruhende und schwach teilungsaktive Zellen, erfolgreich transfiziert werden. Ferner wird durch die Verwendung der Pufferlösung der Einsatz von Hochspannungspulsen mit einer Feldstärke von mindestens 2000 V/cm möglich, was die direkte Transfektion von DNA-Molekülen in den Zellkern primärer tierischer und menschlicher Zellen ermöglicht. Entscheidend für die vorteilhaften Eigenschaften des erfindungsgemäßen Puffers ist dabei die Kombination einer hohen Pufferkapazität mit einer hohen Ionenstärke. Die Pufferkapazität β beschreibt die Menge eines Proteolyten, die erforderlich ist, den pH-Wert einer Pufferlösung um eine pH-Einheit zu ändern (β = dC * dpH⁻¹ mit dC = Zusatz an Säure oder Base und dpH = Änderung des pH-Wertes). Die Ionenstärke I einer Lösung lässt sich mit Hilfe der Formel I = 0,5 Σcᵢ * zᵢ² (cᵢ = lonenkonzentration in mol/l, zᵢ = Ladung der Ionen) errechnen. Zur Berechnung der Ionenstärken der in dieser Anmeldung angegebenen Puffersubstanzen wurde das Programm "Java Buffer Calculator" (Twigger & Beynon, 1998) verwendet. In diesem Rahmen kann der Puffer in seiner Zusammensetzung für die unterschiedlichen Zelltypen und Erfordernisse optimiert werden.

In einer bevorzugten Ausführungsform ist vorgesehen, dass die Pufferlösung eine Pufferkapazität zwischen 22 und 80 mmol * I⁻¹ * pH⁻¹, vorzugsweise zwischen 40 und 70 mmol * I⁻¹ * pH⁻¹, aufweist. Hinsichtlich der Ionenstärke hat sich ein Bereich zwischen 200 und 500 mmol * I⁻¹, vorzugsweise zwischen 250 und 400 mmol * I⁻¹, als besonders vorteilhaft erwiesen. In der Regel liegt also in Abhängigkeit vom Zelltyp und den weiteren Versuchsbedingungen ein Optimum in Bezug auf Pufferkapazität und Ionenstärke vor, wobei eine Wechselwirkung zwischen diesen beiden Parametern besteht.

In einer weiteren bevorzugten Ausführungsform ist vorgesehen, dass die Pufferlösung zumindest 10 mmol * I⁻¹ Magnesium-lonen (Mg²⁺), vorzugsweise 15 bis 20 mmol * I⁻¹ Magnesium-Ionen, enthält. Entgegen der bisherigen Erkenntnisse hat sich überraschenderweise herausgestellt, dass Mg²⁺-lonen in dem erfindungsgemäßen Puffer auch in höheren Konzentrationen eine Steigerung der Transfektionsrate bewirken können und zwar in einem deutlich höheren Maße, als dies durch die gleichzeitige geringfügige Erhöhung der Ionenstärke zu erklären wäre. Dabei kann der Puffer Magnesiumchlorid (MgCl₂) und/oder Magnesiumsulfat (MgSO₄) enthalten.

Der erfindungsgemäße Puffer enthält im Vergleich zum Zytoplasma der Zellen eine geringere Konzentration an Kalium-Ionen (K⁺) und eine höhere Konzentration an Natrium-Ionen (Na⁺). Der erfindungsgemäße Puffer ist also nicht an das intrazelluläre Na⁺/K⁺-Verhältnis angepasst, sondern es liegen diesbezüglich vielmehr "extrazelluläre" Verhältnisse vor. Überraschenderweise hat dies aber keine negativen Auswirkungen auf die Zellen, sondern es kommt dennoch zu einer deutlichen Steigerung der Transfektions- und Zellüberlebensrate.

Es ist vorteilhafterweise vorgesehen, dass der erfindungsgemäße Puffer als Puffersubstanz HEPES und/oder Na₂HPO₄/NaH₂PO₄ enthält. Es können aber beispielsweise auch Tris/HCl oder K₂HPO₄/KH₂PO₄ als Puffersubstanzen verwendet werden. Ferner können auch zusätzliche Bestandteile, beispielsweise Natriumchlorid, Natriumsuccinat, Mannitol, Glucose, Natriumlactobionat und/oder Peptide, in dem Puffer enthalten sein.

Als Beispiele für besonders vorteilhafte Zusammensetzungen der erfindungsgemäßen Pufferlösungen werden im folgenden sechs Gruppen von Puffersystemen genannt, welche jeweils auf unterschiedliche Zelltypen optimiert sind. Es sind aber im Rahmen der Erfindung auch weitere Zusammensetzungen möglich, so dass diese Beispiele nicht als Einschränkung zu verstehen sind.
1) 4 - 6 mM KCl, 10 - 20 mM MgCl₂ und 120 - 160 mM Na₂HPO₄/NaH₂PO₄ (pH 7,2)
2) 4 - 6 mM KCl, 10 - 20 mM MgCl₂, 5 - 25 mM HEPES und 120 -160 mM Na₂HPO₄/NaH₂PO₄ (pH 7,2)
3) 4 - 6 mM KCl, 10 - 20 mM MgCl₂, 50 - 160 mM Na₂HPO₄/NaH₂PO₄ (pH 7,2) und 5 -100 mM Natriumlactobionat oder 5 - 100 mM Mannitol oder 5 - 100 mM Natriumsuccinat oder 5 -100 mM Natriumchlorid
4) 4 - 6 mM KCl, 10 - 20 mM MgCl₂, 5 - 25 mM HEPES, 50 - 160 mM Na₂HPO₄/NaH₂PO₄ (pH 7,2) und 5 - 100 mM Natriumlactobionat oder 5 - 100 mM Mannitol oder 5 - 100 mM Natriumsuccinat oder 5 - 100 mM Natriumchlorid
5) 4 - 6 mM KCl, 10 - 20 mM MgCl₂, 80 - 100 mM NaCl, 8 - 12 mM Glucose, 0,3 - 0,5 mM Ca(NO₃)₂, 20 - 25 mM HEPES und 50 - 100 mM Tris/HCl oder 30 - 50 mM Na₂HPO₄/NaH₂PO₄ (pH 7,2)
6) 0,1 - 3,0 mM MgCl₂, 50 - 200 mM K₂HPO₄/KH₂PO₄ (pH 6,5) und/oder 1 - 50 mM Mannitol und/oder 1 - 50 mM Natriumsuccinat

Die Aufgabe wird ferner durch ein Verfahren zum Einbringen von biologisch aktiven Molekülen in tierische oder menschliche Zellen mittels elektrischen Stroms gelöst, welches das Suspendieren der Zellen und das Lösen der biologisch aktiven Moleküle in einer Pufferlösung, die bei einer Änderung des pH-Wertes von pH 7 nach pH 8 und einer Temperatur von 25 °C eine Pufferkapazität von mindestens 20 mmol * I⁻¹ * pH⁻¹ und eine Ionenstärke von zumindest 200 mmol * I⁻¹ aufweist, und das Anlegen einer elektrischen Spannung an die Suspension umfasst. Mit Hilfe dieses Verfahrens ist das Einbringen biologisch aktiver Moleküle in tierische und menschliche Zellen mittels Elektroporation mit Transfektionseffizienzen von bis zu 93% bei gleichzeitig geringer Zellmortalität möglich. Es können dabei verschiedenen Zelltypen, insbesondere auch ruhende und schwach teilungsaktive Zellen, erfolgreich transfiziert werden.

Weitere vorteilhafte Ausgestaltungen des erfindungsgemäßen Verfahrens in Bezug auf die Zusammensetzung der Pufferlösung sind in den Unteransprüchen angegeben und der voranstehenden Beschreibung zu entnehmen.

Die erfindungsgemäße Pufferlösung eignet sich besonders zur Durchführung eines Elektroporationsverfahrens, bei der das Einbringen der biologisch aktiven Moleküle in die Zellen durch einen Spannungsimpuls mit einer Feldstärke von 2 bis 10 kV*cm⁻¹ und einer Dauer von 10 bis 200 µs sowie einer Stromdichte von mindestens 2 A*cm⁻² erreicht wird. Durch den Hochspannungspuls wird dabei die direkte Transfektion von DNA in den Zellkern tierischer und menschlicher Zellen ermöglicht, wobei durch die Kürze des Pulses eine irreversible Membranschädigung vermieden wird. Die hohe lonenstärke bzw. Leitfähigkeit des Puffers führt hierbei aufgrund der Kürze des Hochspannungspulses auch nicht zu einer unvorteilhaften Erhitzung der Lösung, so dass neben teilungsaktiven auch ruhende oder schwach teilungsaktive Zellen mit hoher Effizienz und geringer Mortalität transfiziert werden können. Das Zusammenwirken von hoher Ionenstärke des Puffers und kurzen Hochspannungspulsen bewirkt vielmehr eine effiziente Porenöffnung, wobei die starke Pufferwirkung auch extreme Schwankungen des pH-Wertes ausgleichen kann.

Auch die Anwendung eines auf den genannten Hochspannungsimpuls ohne Unterbrechung folgenden Stromflusses mit einer Stromdichte von 2 bis 14 A*cm⁻², vorzugsweise bis 5 A*cm⁻², und einer Dauer von 1 bis 100 ms, vorzugsweise bis 50 ms, wird durch die Verwendung des erfindungsgemäßen Puffers in dem Verfahren in besonders vorteilhafter Weise ermöglicht. Die hohe Pufferkapazität der erfindungsgemäßen Pufferlösung verringert dabei während des lang anhaltenden zweiten Pulses, der zur Elektrophorese der DNA beiträgt, eine Änderung des pH-Wertes in der Nähe der Elektroden, so dass die Zellmortalität wirksam verringert werden kann.

Durch das erfindungsgemäße Verfahren wird also in vorteilhafter Weise die Transfektion der biologisch aktiven Moleküle mittels des elektrischen Stroms in den Zellkern der tierischen oder menschlichen Zellen optimiert. Dabei können Nukleinsäuren, Proteine oder Peptide in ruhende oder teilungsaktive, tierische und menschliche Zellen mit hoher Effizienz eingebracht werden. Die erfindungsgemäße Pufferlösung begünstigt durch die Unterstützung von Hochspannungspulsen auch das Einbringen von Nukleinsäuren, Proteinen oder Peptiden in primäre Zellen.

In der Pufferlösung können die Nukleinsäuren auch im Komplex oder in Verbindung mit Peptiden, Proteinen oder anderen biologisch aktiven Molekülen vorliegen.

Das erfindungsgemäße Verfahren eignet sich insbesondere auch für die Transfektion von primären menschlichen Blutzellen, pluripotenten Vorläuferzellen des menschlichen Blutes, primären humanen Fibroblasten und Endothelzellen, sowie Muskelzellen oder Melanozyten.

Die nach dem erfindungsgemäßen Verfahren hergestellten Zellen eignen sich in besonders vorteilhafter Weise für diagnostische und/oder analytische Verfahren sowie zur Herstellung von Arzneimitteln zur ex-vivo Gentherapie.

In Tabelle 1 werden konkrete Zusammensetzungen erfindungsgemäßer Pufferlösungen genannt, welche jeweils für unterschiedliche Anwendungen bzw. Zelltypen optimiert sind und sich als besonders vorteilhaft in Bezug auf hohe Transfektionseffizienzen und Verringerung der Zellmortalität erwiesen haben. Es sind im Rahmen der Erfindung aber auch weitere Zusammensetzungen möglich, so dass auch diese Beispiele nicht als Einschränkung zu verstehen sind.

Anwendungsbeispiele zu einzelnen Pufferlösungen sind im folgenden in Verbindung mit den Figuren näher ausgeführt.

### Beschreibung der Figuren

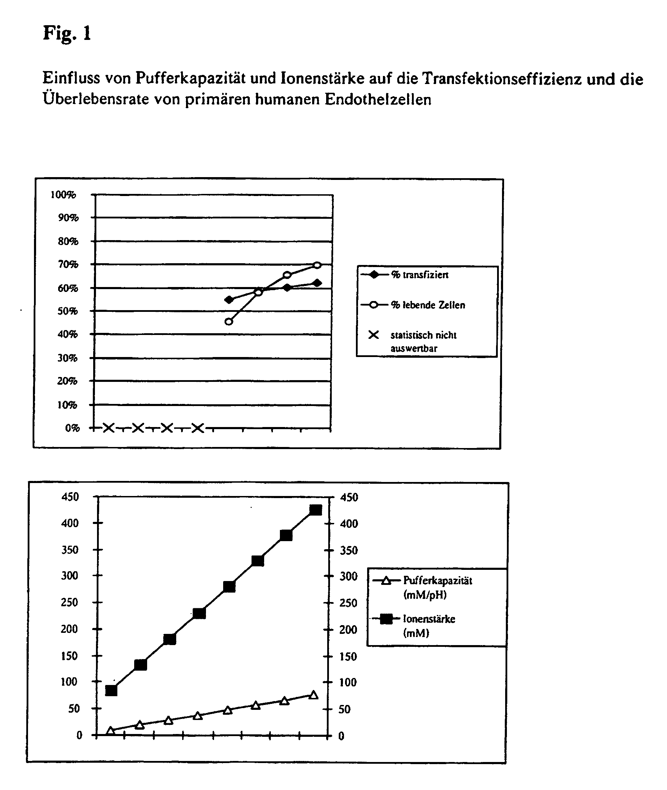

Die Erfindung wird im weiteren anhand der Figuren näher erläutert.
Es zeigt
- Figur 1: eine grafische Darstellung der Transfektionseffizienz und der Überlebensrate bei primären humanen Endothelzellen in Abhängigkeit von Pufferkapazität und Ionenstärke der Pufferlösung,

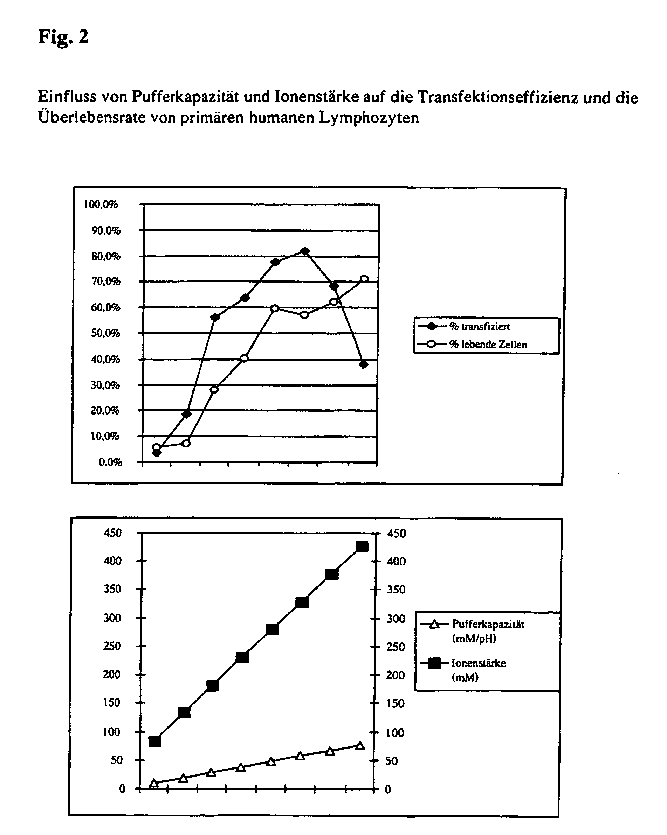

- Figur 2: eine grafische Darstellung der Transfektionseffizienz und der Überlebensrate bei primären humanen Lymphozyten in Abhängigkeit von Pufferkapazität und Ionenstärke der Pufferlösung,

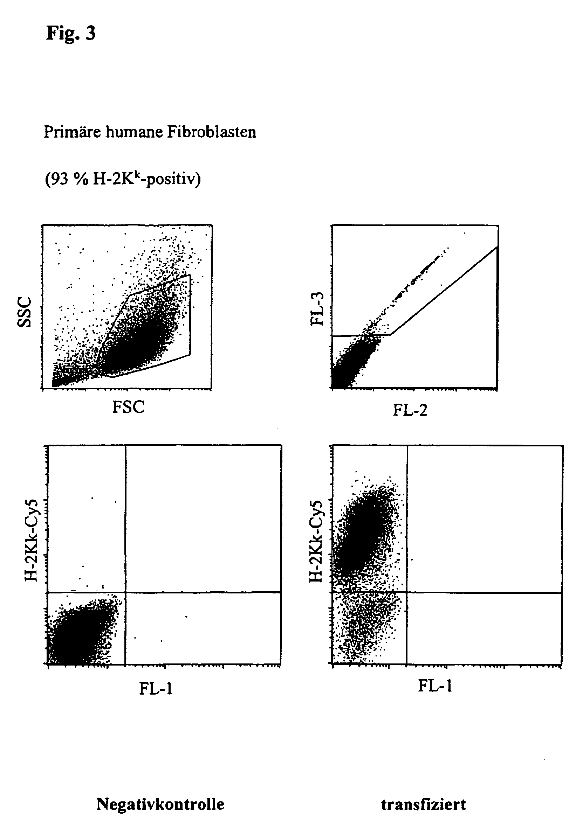

- Figur 3: eine durchfluss-zytometrische Analyse von mit dem H-2K^{k}-Expressionsvektor transfizierten primären humanen Fibroblasten, bei der die Zellen mit einem Cy5-gekoppelten anti-H-2K^{k}-Antikörper inkubiert und mit einem Durchfluss-Zytometer (FACScalibur, Becton Dickinson) analysiert wurden (FL-1, FL-2, FL-3 = Fluoreszenzkanal 1, 2, 3; SSC = sideward scatter, FSC = forward scatter),

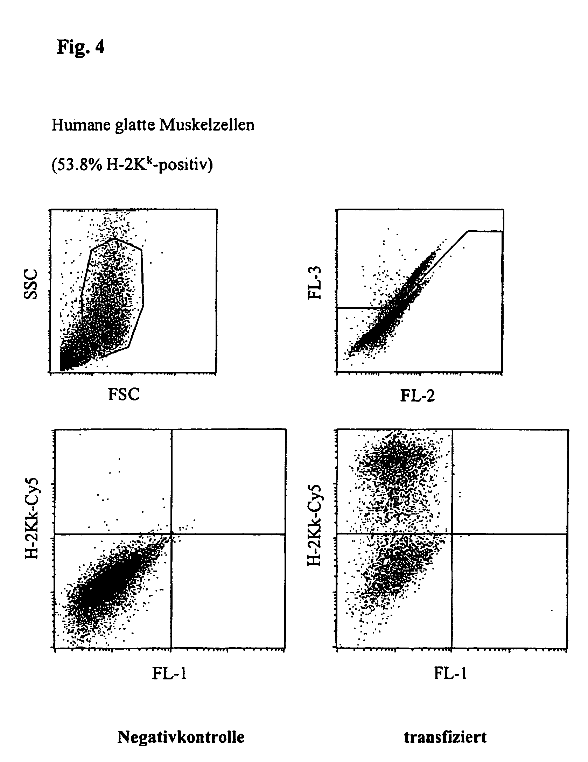

- Figur 4: eine durchfluss-zytometrische Analyse von mit dem H-2K^{k}-Expressionsvektor transfizierten humanen glatten Muskelzellen, bei der die Zellen mit einem Cy5-gekoppelten anti-H-2K^{k}-Antikörper inkubiert und mit einem Durchfluss-Zytometer (FACScalibur, Becton Dickinson) analysiert wurden (FL-1, FL-2, FL-3 = Fluoreszenzkanal 1, 2, 3; SSC = sideward scatter, FSC = forward scatter),

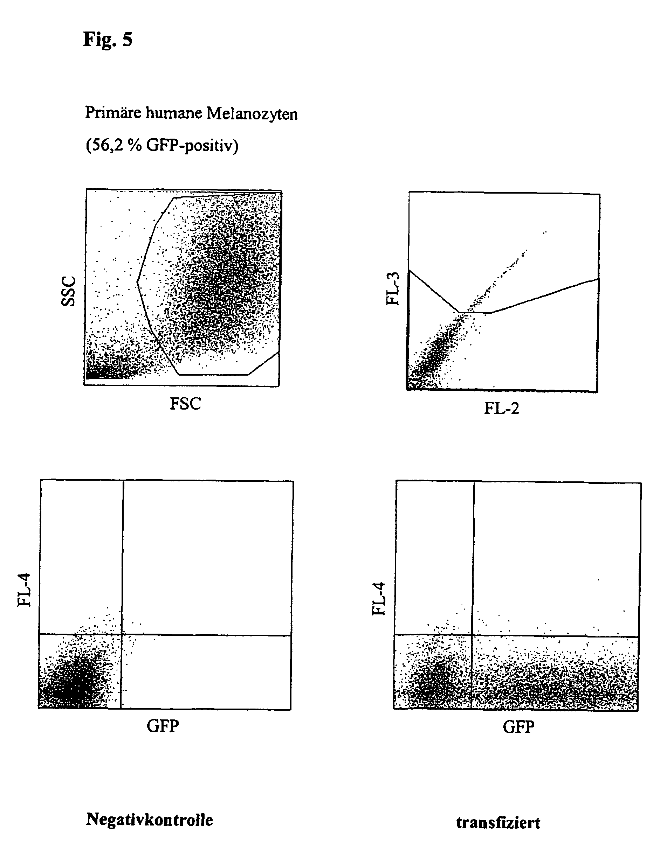

- Figur 5: eine durchfluss-zytometrische Analyse von mit dem GFP-Expressionsvektor transfizierten primären humanen Melanozyten, bei der die Zellen mit einem Durchfluss-Zytometer (FACScalibur, Becton Dickinson) analysiert wurden (FL-2, FL-3, FL-4 = Fluoreszenzkanal 2, 3, 4; SSC = sideward scatter, FSC = forward scatter), und

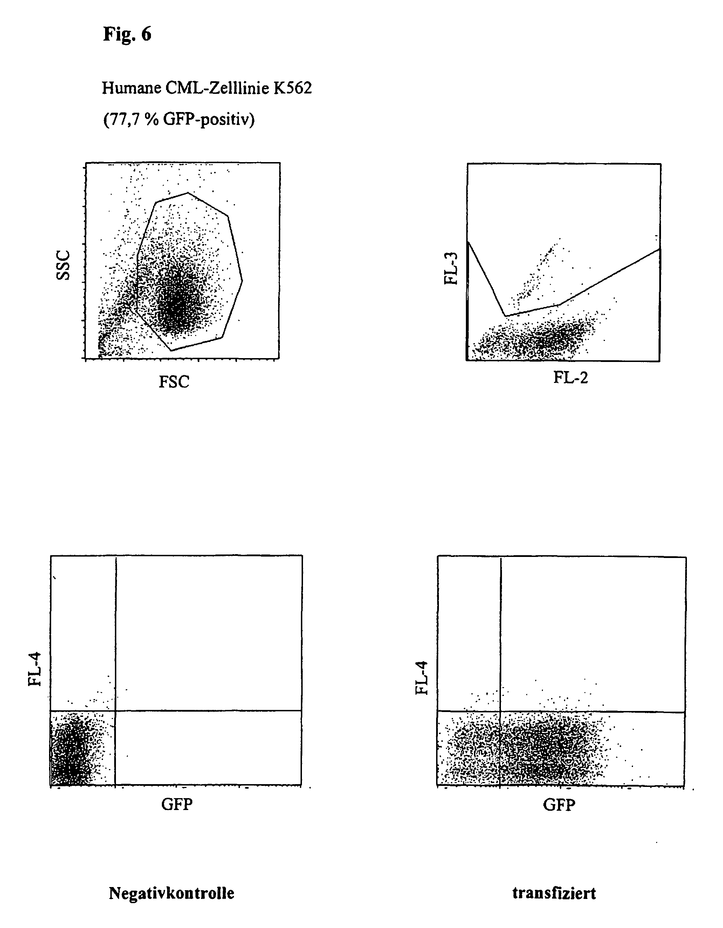

- Figur 6: eine durchfluss-zytometrische Analyse der mit dem GFP-Expressionsvektor transfizierten humanen CML-Zelllinie K562, bei der die Zellen mit einem Durchfluss-Zytometer (FACScalibur, Becton Dickinson) analysiert wurden (FL-2, FL-3, FL-4 = Fluoreszenzkanal 2, 3, 4; SSC = sideward scatter, FSC = forward scatter),

### Beispiele

Die nachstehenden Beispiele veranschaulichen die Erfindung, sind jedoch nicht begrenzend aufzufassen.

### Beispiel 1

### Transfektionseffizienz und Überlebensrate von primären humanen Endothelzellen

Um die Transfektions- und Überlebensrate von Zellen bei der Elektrotransfektion in Abhängigkeit von der Pufferkapazität und der Ionenstärke zu untersuchen, wurden primäre humane Endothelzellen mit einem Vektor, der für die schwere Kette des Maus MHC Klasse I Proteins H-2K^{k} kodiert, transfiziert.
Jeweils 7 x 10⁵ Zellen wurden mit jeweils 5 µg Vektor-DNA mit dem jeweiligen Elektrotransfektionspuffer bei Raumtemperatur in eine Küvette mit 2 mm Elektrodenabstand gegeben und durch einen Puls von 1000 V und 100 µs Dauer transfiziert. Zusätzlich wurden Vergleichswerte mit PBS (Phosphate Buffered Saline: 10 mM Natriumphosphat, pH 7,2 + 137 mM NaCl, Ionenstärke = 161,5 mM, Pufferkapazität = 4,8 mM/pH) ermittelt. Unmittelbar nach der Elektrotransfektion wurden die Zellen mit 400 µl Kulturmedium aus der Küvette gespült, für 10 Minuten bei 37°C inkubiert und dann in eine Kulturschale mit vorgewärmtem Medium überführt. Nach 6 h Inkubation wurden die Zellen mit einem Cy5-gekoppeltem anti-H-2K^{k}-Antikörper und mit einem Durchfluß-Zytometer (FACScalibur, Becton Dickinson) analysiert.
Wie aus Figur 1 deutlich wird, erhöhen sich sowohl die Transfektionseffizienzen als auch die Übenebensraten mit steigender Pufferkapazität und Ionenstärke. Dabei konnten jeweils deutlich bessere Werte erzielt werden als mit der Vergleichlösung PBS, in der eine extrem hohe Mortalitätsrate auftrat. Auch bei einigen Pufferlösungen war die Mortalitätsrate so hoch, dass diese Werte statistisch nicht auswertbar waren.

### Beispiel 2

### Transfektionseffizienz und Überlebensrate von primären humanen Lymphozyten

Desweiteren wurde die Transfektions- und Überlebensrate von primären humanen Lymphozyten bei der Elektrotransfektion in Abhängigkeit von der Pufferkapazität und der Ionenstärke untersucht.
Dazu wurden jeweils 5 x 10⁶ Zellen mit je 5 µg H-2K^{k}-Expressionsvektor-DNA in den jeweiligen Puffern bei Raumtemperatur in eine Küvette mit 2 mm Elektrodenabstand gegeben und durch einen Puls von 1000 V und 100 µs, gefolgt von einem Stromfluss mit einer Stromdichte von 4 A*cm⁻² und 40 ms elektrotransfiziert. Zusätzlich wurden Vergleichswerte unter Verwendung von PBS (Ionenstärke = 161,5 mM, Pufferkapazität = 4,8 mM/PH) ermittelt. Die Analyse erfolgte nach 16 Stunden.
Wie in Figur 2 zu erkennen, wird hier deutlich, dass zwar die Übenebensraten der Zellen mit steigender Pufferkapazität und Ionenstärke der verwendeten Pufferlösungen ansteigen, sich aber für die Transfektionseffizienzen ein Maximum ergibt. Es konnten teilweise deutlich bessere Werte erzielt werden als mit der Vergleichlösung (PBS: 40,1 % transfizierte Zellen, 38,3 % lebende Zellen). Auch hier läßt sich die Effizienz der Elektrotransfektion also durch die Wahl der geeigneten erfindungsgemäßen Pufferlösung im Vergleich zu herkömmlichen Lösungen, wie z.B. Medium oder PBS, deutlich steigern.

### Beispiel 3

### Transfektion von primären humanen Fibroblasten

Figur 3 zeigt eine FACScan-Analyse von primären humanen Fibroblasten, die mit dem H-2K^{k}-Expressionsvektor transfiziert wurden. 5 x 10⁵ Zellen wurden mit 5 µg Vektor-DNA in Puffer 6 aus Tabelle 1 bei Raumtemperatur in eine Küvette mit 2 mm Elektrodenabstand gegeben und durch einen Puls von 1000 V und 100 µs, gefolgt von einem Stromfluss mit einer Stromdichte von 6 A*cm⁻² und 33 ms transfiziert. Nach 5 h Inkubation wurden die Zellen mit einem Cy5-gekoppeltem anti-H-2K^{k}-Antikörper inkubiert und mit einem Durchfluß-Zytometer (FACScalibur, Becton Dickinson) analysiert. Durch Färbung mit Propidiumjodid wurde die Anzahl toter Zellen bestimmt. 93 % der lebenden Zellen exprimierten das H-2K^{k}-Antigen, was einer sehr hohen Transfektionseffizienz entspricht.

### Beispiel 4

### Transfektion von humanen glatten Muskelzellen

Figur 4 zeigt eine FACScan-Analyse von humanen glatten Muskelzellen, die mit dem H-2K^{k}-Expressionsvektor transfiziert wurden. 5 x 10⁵ Zellen wurden mit 5 pg Vektor-DNA in Puffer 6 aus Tabelle 1 bei Raumtemperatur in eine Küvette mit 2 mm Elektrodenabstand gegeben und durch einen Puls von 1000V und 100 µs, gefolgt von einem Stromfluss mit einer Stromdichte von 5,6 A*cm⁻² und 40 ms transfiziert. Nach 13,5 h Inkubation wurden die Zellen mit einem Cy5-gekoppeltem anti-H-2K^{k}-Antikörper inkubiert und mit einem Durchfluß-Zytometer (FACScalibur, Becton Dickinson) analysiert. Durch Färbung mit Propidiumjodid wurde die Anzahl toter Zellen bestimmt. 53,8 % der lebenden Zellen exprimierten das H-2K^{k}-Antigen, was ebenfalls einer hohen Transfektionseffizienz entspricht.

### Beispiel 5

### Transfektion von primären humanen Melanozyten

Figur 5 zeigt eine FACScan-Analyse von primären humanen Melanozyten, die mit einem GFP-Expressionsvektor transfiziert wurden. 5 x 10⁵ Zellen wurden mit 5 µg pEGFP-C1 - Vektor (Clontech Lab.) in Puffer 40 gemäß Tabelle 1 bei Raumtemperatur in eine Küvette mit 2 mm Elektrodenabstand gegeben und durch einen Puls von 1000V und 100 µs, gefolgt von einem Stromfluss mit einer Stromdichte von 6 A*cm⁻² und 33 ms transfiziert. Nach 24 h Inkubation wurden die Zellen mit einem Durchfluß-Zytometer (FACScalibur, Becton Dickinson) analysiert. Durch Färbung mit Propidiumjodid wurde die Anzahl toter Zellen bestimmt. 56,2 % der lebenden Zellen exprimierten das GFP, was ebenfalls einer hohen Transfektionseffizienz entspricht.

### Beispiel 6

### Transfektion von humanen CML-Zelllinie K562

Figur 6 zeigt eine FACScan-Analyse der humanen CML-Zelllinie K562, die mit einem GFP-Expressionsvektor transfiziert wurden. 5 x 10⁵ Zellen wurden mit 5 µg pEGFP-C1 - Vektor (Clontech Lab.) in Puffer 42 gemäß Tabelle 1 bei Raumtemperatur in eine Küvette mit 2 mm Elektrodenabstand gegeben und durch einen Puls von 1000V und 70 µs, gefolgt von einem Stromfluss mit einer Stromdichte von 4 A*cm⁻² und 10 ms transfiziert. Nach 24 h Inkubation wurden die Zellen mit einem Durchfluß-Zytometer (FACScalibur, Becton Dickinson) analysiert. Durch Färbung mit Propidiumjodid wurde die Anzahl toter Zellen bestimmt. 77,7 % der lebenden Zellen exprimierten das GFP, was ebenfalls einer hohen Transfektionseffizienz entspricht.

### Liste der verwendeten Abkürzungen

Außer den im Duden gebräuchlichen, wurden folgende Abkürzungen verwendet:
- A: Ampere
- APC: Allphycocyanin
- CD: cluster of differentiation
- cm: Zentimeter
- DNA: Desoxyribonukleinsäure
- FACScan: Fluorescence activated cell scanning
- FCS: foetales Kälberserum
- FL: Fluroeszenz
- FSC: Forwardscatter
- HEPES: N-(2-Hydroxyethyl)-Piperazin-N'-(2-Ethansulfonsäure)
- ml: Milliliter
- mM: millimolar
- msec: Millisekunde
- PBS: Phosphat buffered Saline
- PE: Phycoerythrin
- PerCP: Peridinin Chlorophyll Protein
- pH: negativer dekadischer Logarithums der Wasserstoff-Ionenkonzentration
- RNA: Ribonukleinsäure
- RPMI: Rosewell Park Memorial Institute
- SSC: Sidewardscatter
- Tris: Tris-(Hydroxymethyl)-Aminoethan-Hydrochlorid
- µg: Mikrogramm
- µl: Mikroliter
- µsec: Mikrosekunde
- V: Volt

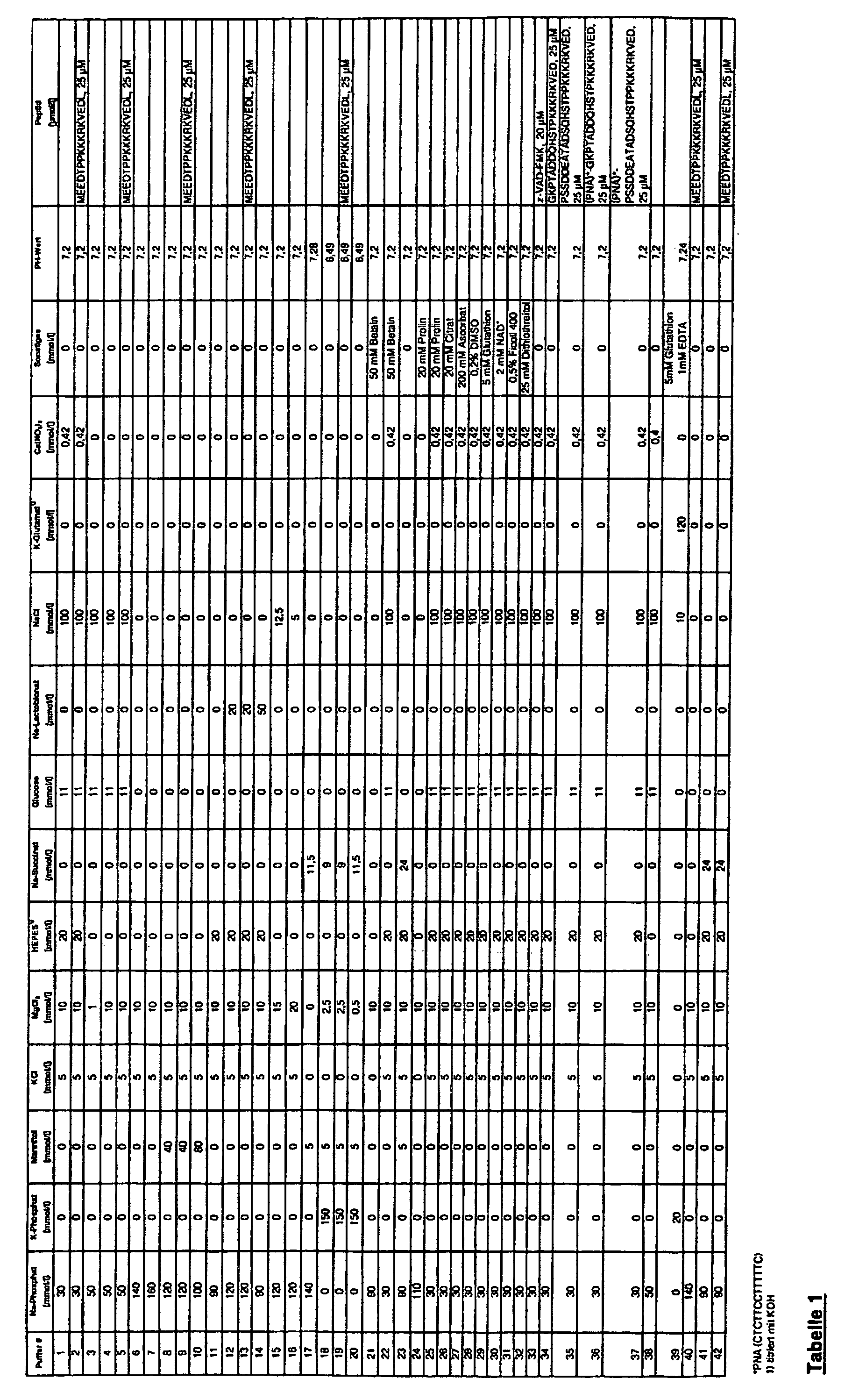

## Patentansprüche

1. Pufferlösung zum Suspendieren tierischer oder menschlicher Zellen und Lösen biologisch aktiver Moleküle zum Zwecke des Einbringens dieser biologisch aktiven Moleküle in die Zellen mittels elektrischen Stroms, die bei einer Änderung des pH-Wertes von pH 7 nach pH 8 und einer Temperatur von 25 °C eine Pufferkapazität von mindestens 20 mmol * I⁻¹ * pH⁻¹ und eine lonenstärke von zumindest 200 mmol * I⁻¹ aufweist, und bei der die Konzentration an Kalium-Ionen (K⁺) 2 bis 6 mmol * I⁻¹ K⁺ und die Konzentration an Natrium-Ionen (Na⁺) 100 bis 150 mmol * I⁻¹ Na⁺ beträgt.

2. Pufferlösung nach Anspruch 1, **dadurch gekennzeichnet, dass** diese eine Pufferkapazität (pH 7-8, 25 °C) zwischen 22 und 80 mmol * I⁻¹ * pH⁻¹ aufweist.

3. Pufferlösung nach Anspruch 1 oder 2, **dadurch gekennzeichnet, dass** diese eine Pufferkapazität (pH 7-8, 25 °C) zwischen 40 und 70 mmol * I⁻ ¹ * pH⁻¹ aufweist.

4. Pufferlösung nach Anspruch 1, 2 oder 3, **dadurch gekennzeichnet, dass** diese eine Ionenstärke zwischen 200 und 500 mmol * I⁻¹ aufweist.

5. Pufferlösung nach Anspruch 1, 2, 3 oder 4, **dadurch gekennzeichnet, dass** diese eine Ionenstärke zwischen 250 und 400 mmol * I⁻¹ aufweist.

6. Pufferlösung nach einem der Ansprüche 1 bis 5, **dadurch gekennzeichnet, dass** diese zumindest 10 mmol * I⁻¹ Magnesium-Ionen (Mg²⁺) enthält.

7. Pufferlösung nach Anspruch 6, **dadurch gekennzeichnet, dass** diese 15 bis 20 mmol * I⁻¹ Magnesium-lonen enthält.

8. Pufferlösung nach einem der Ansprüche 1 bis 7, **dadurch gekennzeichnet, dass** diese Magnesiumchlorid (MgCl₂) und/oder Magnesiumsulfat (MgSO₄) enthält.

9. Pufferlösung nach einem der Ansprüche 1 bis 8, **dadurch gekennzeichnet, dass** als Puffersubstanz HEPES und/oder Na₂HPO₄/NaH₂PO₄ und/oder Tris/HCl und/oder K₂HPO₄/KH₂PO₄ enthalten ist.

10. Pufferlösung nach einem der Ansprüche 1 bis 9, **dadurch gekennzeichnet, dass** zusätzliche Bestandteile, beispielsweise Natriumchlorid, Natriumsuccinat, Mannitol, Glucose, Natriumlactobionat und/oder Peptide, in der Pufferlösung enthalten sind.

11. Pufferlösung nach einem der Ansprüche 1 bis 10, **dadurch gekennzeichnet, dass** diese 4 - 6 mM KCl, 10 - 20 mM MgCl₂ und 120 - 160 mM Na₂HPO₄/NaH₂PO₄ (pH 7,2) enthält.

12. Pufferlösung nach einem der Ansprüche 1 bis 10, **dadurch gekennzeichnet, dass** diese 4 - 6 mM KCl, 10 - 20 mM MgCl₂, 5 - 25 mM HEPES und 120 -160 mM Na₂HPO₄/NaH₂PO₄ (pH 7,2) enthält.

13. Pufferlösung nach einem der Ansprüche 1 bis 10, **dadurch gekennzeichnet, dass** diese 4 - 6 mM KCl, 10 - 20 mM MgCl₂, 50 -160 mM Na₂HPO₄/NaH₂PO₄ (pH 7,2) und 5 - 100 mM Natriumlactobionat oder 5 - 100 mM Mannitol oder 5 - 100 mM Natriumsuccinat oder 5 - 100 mM Natriumchlorid enthält.

14. Pufferlösung nach einem der Ansprüche 1 bis 10, **dadurch gekennzeichnet, dass** diese 4 - 6 mM KCl, 10 - 20 mM MgCl₂, 5 - 25 mM HEPES, 50 -160 mM Na₂HPO₄/NaH₂PO₄ (pH 7,2) und 5 - 100 mM Natriumlactobionat oder 5 - 100 mM Mannitol oder 5 - 100 mM Natriumsuccinat oder 5 -100 mM Natriumchlorid enthält.

15. Pufferlösung nach einem der Ansprüche 1 bis 10, **dadurch gekennzeichnet, dass** diese 4 - 6 mM KCl, 10 - 20 mM MgCl₂, 80 - 100 mM NaCl, 8 - 12 mM Glucose, 0,3 - 0,5 mM Ca(NO₃)₂, 20 - 25 mM HEPES und 50 -100 mM Tris/HCl oder 30 - 50 mM Na₂HPO₄/NaH₂PO₄ (pH 7,2) enthält.

16. Pufferlösung nach einem der Ansprüche 1 bis 10, **dadurch gekennzeichnet, dass** diese 0,1 - 3,0 mM MgCl₂, 50 - 200 mM K₂HPO₄/KH₂PO₄ (pH 6,5) und/oder 1 - 50 mM Mannitol und/oder 1 - 50 mM Natriumsuccinat enthält.

17. Verfahren zum Einbringen von biologisch aktiven Molekülen in tierische oder menschliche Zellen mittels elektrischen Stroms, umfassend:
i. Suspendieren der Zellen und Lösen der biologisch aktiven Moleküle in einer Pufferlösung, die bei einer Änderung des pH-Wertes von pH 7 nach pH 8 und einer Temperatur von 25 °C eine Pufferkapazität von mindestens 20 mmol * I⁻¹ * pH⁻¹ und eine lonenstärke von zumindest 200 mmol * I⁻¹ aufweist und
ii. Anlegen einer elektrischen Spannung an die Suspension.

18. Verfahren nach Anspruch 17, wobei die Pufferlösung eine Pufferkapazität (pH 7-8, 25 °C) zwischen 22 und 80 mmol * I⁻¹ * pH⁻¹ aufweist.

19. Verfahren nach Anspruch 17 oder 18, wobei die Pufferlösung eine Pufferkapazität (pH 7-8, 25 °C) zwischen 40 und 70 mmol * I⁻¹ * pH⁻¹ aufweist.

20. Verfahren nach Anspruch 17, 18 oder 19, wobei die Pufferlösung eine Ionenstärke zwischen 200 und 500 mmol * I⁻¹ aufweist.

21. Verfahren nach einem der Ansprüche 17 bis 20, wobei die Pufferlösung eine lonenstärke zwischen 250 und 400 mmol * I⁻¹ aufweist.

22. Verfahren nach einem der Ansprüche 17 bis 21, wobei die Pufferlösung zumindest 10 mmol * I⁻¹ Magnesium-lonen (Mg²⁺) enthält.

23. Verfahren nach Anspruch 22, wobei die Pufferlösung 15 bis 20 mmol * I⁻¹ Magnesium-Ionen enthält.

24. Verfahren nach einem der Ansprüche 17 bis 23, wobei die Pufferlösung Magnesiumchlorid (MgCl₂) und/oder Magnesiumsulfat (MgSO₄) enthält.

25. Verfahren nach einem der Ansprüche 17 bis 24, wobei die Pufferlösung im Vergleich zum Zytoplasma der Zellen eine geringere Konzentration an Kalium-Ionen (K⁺), vorzugsweise 2 bis 6 mmol * I⁻¹ K⁺, und eine höhere Konzentration an Natrium-Ionen (Na⁺), vorzugsweise 100 bis 150 mmol * l⁻¹ Na⁺, aufweist.

26. Verfahren nach einem der Ansprüche 17 bis 25, wobei als Puffersubstanz HEPES und/oder Na₂HPO₄/NaH₂PO₄ und/oder Tris/HCl und/oder K₂HPO₄/KH₂PO₄ enthalten ist.

27. Verfahren nach einem der Ansprüche 17 bis 26, wobei zusätzliche Bestandteile, beispielsweise Natriumchlorid, Natriumsuccinat, Mannitol, Glucose, Natriumlactobionat und/oder Peptide, in der Pufferlösung enthalten sind.

28. Verfahren nach einem der Ansprüche 17 bis 27, wobei die Pufferlösung 4 - 6 mM KCl, 10 - 20 mM MgCl₂ und 120 - 160 mM Na₂HPO₄/NaH₂PO₄ (pH 7,2) enthält.

29. Verfahren nach einem der Ansprüche 17 bis 27, wobei die Pufferlösung 4 - 6 mM KCl, 10 - 20 mM MgCl₂, 5 - 25 mM HEPES und 120 - 160 mM Na₂HPO₄/NaH₂PO₄ (pH 7,2) enthält.

30. Verfahren nach einem der Ansprüche 17 bis 27, wobei die Pufferlösung 4 - 6 mM KCl, 10 - 20 mM MgCl₂, 50 - 160 mM Na₂HPO₄/NaH₂PO₄ (pH 7,2) und 5 - 100 mM Natriumlactobionat oder 5 -100 mM Mannitol oder 5 -100 mM Natriumsuccinat oder 5 -100 mM Natriumchlorid enthält.

31. Verfahren nach einem der Ansprüche 17 bis 27, wobei die Pufferlösung 4 - 6 mM KCl, 10 - 20 mM MgCl₂, 5 - 25 mM HEPES, 50 - 160 mM Na₂HPO₄/NaH₂PO₄ (pH 7,2) und 5 - 100 mM Natriumlactobionat oder 5 - 100 mM Mannitol oder 5 - 100 mM Natriumsuccinat oder 5 - 100 mM Natriumchlorid enthält.

32. Verfahren nach einem der Ansprüche 17 bis 27, wobei die Pufferlösung 4 - 6 mM KCl, 10 - 20 mM MgCl₂, 80 - 100 mM NaCl, 8 - 12 mM Glucose, 0,3 - 0,5 mM Ca(NO₃)₂, 20 - 25 mM. HEPES und 50 - 100 mM Tris/HCl oder 30 - 50 mM Na₂HPO₄/NaH₂PO₄ (pH 7,2) enthält.

33. Verfahren nach einem der Ansprüche 17 bis 27, wobei die Pufferlösung 0,1 - 3,0 mM MgCl₂, 50 - 200 mM K₂HPO₄/KH₂PO₄ (pH 6,5) und/oder 1 - 50 mM Mannitol und/oder 1 - 50 mM Natriumsuccinat enthält.

34. Verfahren nach einem der Ansprüche 17 bis 33, wobei das Einbringen der biologisch aktiven Moleküle in die Zellen durch einen Spannungsimpuls mit einer Feldstärke von 2 bis 10 kV*cm⁻¹ und einer Dauer von 10 bis 200 µs sowie einer Stromdichte von mindestens 2 A*cm⁻² erreicht wird.

35. Verfahren nach Anspruch 34, wobei das Einbringen der biologisch aktiven Moleküle in die Zellen durch einen auf den Impuls ohne Unterbrechung folgenden Stromfluss mit einer Stromdichte von 2 A*cm⁻² bis 14 A*cm⁻², vorzugsweise bis 5 A*cm⁻², und einer Dauer von 1 bis 100 ms, vorzugsweise bis 50 ms, erreicht wird.

36. Verfahren nach Anspruch 34 oder 35, wobei die biologisch aktiven Moleküle mittels des elektrischen Stroms in den Zellkern der tierischen oder menschlichen Zellen transfiziert werden.

37. Verfahren nach einem der Ansprüche 17 bis 36, wobei Nukleinsäuren, Proteine, Peptide oder andere biologisch aktive Moleküle in ruhende oder teilungsaktive, tierische oder menschliche Zellen eingebracht werden.

38. Verfahren nach einem der Ansprüche 17 bis 37, wobei Nukleinsäuren, Proteine oder Peptide in primäre, tierische oder menschliche Zellen eingebracht werden.

39. Verfahren nach Anspruch 37 oder 38, wobei die Nukleinsäuren im Komplex oder in Verbindung mit Peptiden, Proteinen oder anderen biologisch aktiven Molekülen vorliegen.

40. Verfahren nach einem der Ansprüche 17 bis 39, wobei die Zellen primäre menschliche Blutzellen, pluripotente Vorläuferzellen des menschlichen Blutes, humane Fibroblasten, humane Endothelzellen, Muskelzellen oder Melanozyten sind.

## Claims

1. A buffer solution for suspending animal or human cells and for dissolving biologically active molecules in order to introduce said biologically active molecules into the cells using electric current, the buffer solution has a buffer capacity of at least 20 mmol * I⁻¹ * pH⁻¹ and an ionic strength of at least 200 mmol * I⁻¹ at a change in the pH from pH 7 to pH 8 and at a temperature of 25 °C, and contains a concentration of potassium ions (K⁺) between 2 to 6 mmol * I⁻¹ K⁺, and a concentration of sodium ions (Na⁺) between 100 to 150 mmol * I⁻¹ Na⁺.

2. The buffer solution according to claim 1 having a buffer capacity (pH 7-8, 25°C) between 22 and 80 mmol * I⁻¹ * pH⁻¹.

3. The buffer solution according to claim 1 or 2 having a buffer capacity (pH 7-8, 25°C) between 40 and 70 mmol * I⁻¹ * pH⁻¹.

4. The buffer solution according to claim 1, 2 or 3 having an ionic strength between 200 and 500 mmol * I⁻¹.

5. The buffer solution according to claim 1, 2, 3 or 4 having an ionic strength between 250 and 400 mmol * I⁻¹.

6. The buffer solution according to any one of the claims 1 to 5 containing at least 10 mmol * I⁻¹ magnesium ions (Mg²⁺).

7. The buffer solution according to claim 6 containing 15 to 20 mmol * I⁻¹ magnesium ions.

8. The buffer solution according to any one of the claims 1 to 7 containing magnesium chloride (MgCl₂) and/or magnesium sulphate (MgSO₄).

9. The buffer solution according to any one of the claims 1 to 8 containing HEPES and/or Na₂HPO₄/NaH₂PO₄ and/or Tris/HCl and/or K₂HPO₄/KH₂PO₄ as buffer substance.

10. The buffer solution according to any one of the claims 1 to 9 containing additional components, for example, sodium chloride, sodium succinate, mannitol, glucose, sodium lactobionate and/or peptides.

11. The buffer solution according to any one of the claims 1 to 10 containing 4 - 6 mM KCl, 10 - 20 mM MgCl₂ and 120 - 160 mM Na₂HPO₄/NaH₂PO₄ (pH 7.2).

12. The buffer solution according to any one of the claims 1 to 10 containing 4 - 6 mM KCl, 10 - 20 mM MgCl₂, 5 - 25 mM HEPES and 120 - 160 mM Na₂HPO₄/NaH₂PO₄ (pH 7.2).

13. The buffer solution according to any one of the claims 1 to 10 containing 4 - 6 mM KCl, 10 - 20 mM MgCl₂, 50 - 160 mM Na₂HPO₄/NaH₂PO₄ (pH 7.2) and 5 - 100 mM sodium lactobionate or 5 - 100 mM mannitol or 5 - 100 mM sodium succinate or 5 - 100 mM sodium chloride.

14. The buffer solution according to any one of the claims 1 to 10 containing 4 - 6 mM KCl, 10 - 20 mM MgCl₂, 5 - 25 mM HEPES, 50 - 160 mM Na₂HPO₄/NaH₂PO₄ (pH 7.2) and 5 - 100 mM sodium lactobionate or 5 - 100 mM mannitol or 5 - 100 mM sodium succinate or 5 - 100 mM sodium chloride.

15. The buffer solution according to any one of the claims 1 to 10 containing 4-6 mM KCl, 10 - 20 mM MgCl₂, 80 - 100 mM NaCl, 8 - 12 mM glucose, 0.3 - 0.5 mM Ca(NO₃)₂, 20 - 25 mM HEPES and 50 - 100 mM tris/HCl or 30 - 50 mM Na₂HPO₄/NaH₂PO₄ (pH 7.2)

16. The buffer solution according to any one of the claims 1 to 10 containing 0.1 - 3.0 mM MgCl₂, 50 - 200 mM K₂HPO₄/KH₂PO₄ (pH 6.5) and/or 1 - 50 mM mannitol and/or 1 - 50 mM sodium succinate.

17. A method for introducing biologically active molecules into animal or human cells by means of electric current, comprising:
i. suspending the cells and dissolving the biologically active molecules in a buffer solution which has a buffer capacity of at least 20 mmol * I⁻¹ * pH⁻¹ and an ionic strength of at least 200 mmol * I⁻¹ at a change in the pH from pH 7 to pH 8 and at a temperature of 25°C, and
ii. applying an electric voltage to the suspension.

18. The method according to claim 17, wherein the buffer solution has a buffer capacity (pH 7-8, 25°C) between 22 and 80 mmol * I⁻¹ * pH⁻¹.

19. The method according to claim 17 or 18, wherein the buffer solution has a buffer capacity (pH 7-8, 25°C) between 40 and 70 mmol * I⁻¹ * pH⁻¹.

20. The method according to claim 17, 18 or 19, wherein the buffer solution has an ionic strength between 200 and 500 mmol * I⁻¹.

21. The method according to any one of the claims 17 to 20, wherein the buffer solution has an ionic strength between 250 and 400 mmol * I⁻¹.

22. The method according to any one of the claims 17 to 21, wherein the buffer solution contains at least 10 mmol * I⁻¹ magnesium ions (Mg²⁺).

23. The method according to claim 22, wherein the buffer solution contains 15 to 20 mmol * I⁻¹ magnesium ions.

24. The method according to any one of the claims 17 to 23, wherein the buffer solution contains magnesium chloride (MgCl₂) and/or magnesium sulphate (MgSO₄).

25. The method according to any one of the claims 17 to 24, wherein the buffer solution contains a lower concentration of potassium ions (K⁺), preferably 2 to 6 mmol * I⁻¹ K⁺, and a higher concentration of sodium ions (Na⁺), preferably 100 to 150 mmol * I⁻¹ Na⁺, if compared with the cytoplasm of the cells.

26. The method according to any one of the claims 17 to 25, wherein the buffer solution contains HEPES and/or Na₂HPO₄/NaH₂PO₄ and/or Tris/HCl and/or K₂HPO₄/KH₂PO₄ as buffer substance.

27. The method according to any one of the claims 17 to 26, wherein the buffer solution contains additional components, for example, sodium chloride, sodium succinate, mannitol, glucose, sodium lactobionate and/or peptides.

28. The method according to any one of the claims 17 to 27, wherein the buffer solution contains 4 - 6 mM KCl, 10 - 20 mM MgCl₂ and 120 - 160 mM Na₂HPO₄/NaH₂PO₄ (pH 7.2).

29. The method according to any one of the claims 17 to 27, wherein the buffer solution contains 4 - 6 mM KCl, 10 - 20 mM MgCl₂, 5 - 25 mM HEPES and 120 - 160 mM Na₂HPO₄/NaH₂PO₄ (pH 7.2).

30. The method according to any one of the claims 17 to 27, wherein the buffer solution contains 4 - 6 mM KCl, 10 - 20 mM MgCl₂, 50 - 160 mM Na₂HPO₄/NaH₂PO₄ (pH 7.2) and 5 - 100 mM sodium lactobionate or 5 - 100 mM mannitol or 5 - 100 mM sodium succinate or 5 - 100 mM sodium chloride.

31. The method according to any one of the claims 17 to 27, wherein the buffer solution contains 4 - 6 mM KCl, 10 - 20 mM MgCl₂, 5 - 25 mM HEPES, 50 - 160 mM Na₂HPO₄/NaH₂PO₄ (pH 7.2) and 5 - 100 mM sodium lactobionate or 5 - 100 mM mannitol or 5 - 100 mM sodium succinate or 5 - 100 mM sodium chloride.

32. The method according to any one of the claims 17 to 27, wherein the buffer solution contains 4- 6 mM KCl, 10 - 20 mM MgCl₂, 80 - 100 mM NaCl, 8 - 12 mM glucose, 0.3 - 0.5 mM Ca(NO₃)₂, 20 - 25 mM HEPES and 50 -100 mM tris/HCl or 30 - 50 mM Na₂HPO₄/NaH₂PO₄ (pH 7.2)

33. The method according to any one of the claims 17 to 27, wherein the buffer solution contains 0.1 - 3.0 mM MgCl₂, 50 - 200 mM K₂HPO₄/KH₂PO₄ (pH 6.5) and/or 1 - 50 mM mannitol and/or 1 - 50 mM sodium succinate.

34. The method according to any one of the claims 17 to 33, wherein the introduction of the biologically active molecules into the cells is achieved by a voltage pulse having a field strength of up to 2 to 10 kV*cm⁻¹ and a duration of 10 to 200 µs and a current density of at least 2 A*cm⁻².

35. The method according to claim 34, wherein the introduction of the biologically active molecules into the cells is achieved by a current flow following said high-voltage pulse without interruption, having a current density of 2 to 14 A*cm⁻², preferably up to 5 A*cm⁻², and a duration of 1 to 100 ms, preferably up to 50 ms.

36. The method according to claim 34 or 35, wherein the biologically active molecules are transfected into the cell nucleus of animal or human cells by means of electric current.

37. The method according to any one of the claims 17 to 36, wherein nucleic acids, proteins or peptides are introduced into quiescent or dividing animal or human cells.

38. The method according to any one of the claims 17 to 37, wherein nucleic acids, proteins or peptides are introduced into primary animal or human cells.

39. The method according to claim 37 or 38, wherein the nucleic acids are present in complexes or in compounds with peptides, proteins or other biologically active molecules.

40. The method according to any one of the claims 17 to 39, wherein the cells are primary human blood cells, pluripotent precursor cells of human blood, primary human fibroblasts and endothelial cells, as well as muscle cells or melanocytes.

## Revendications

1. Solution tampon pour la mise en suspension de cellules animales ou humaines et pour dissoudre des molécules biologiquement actives à des fins d'introduction de ces molécules biologiquement actives dans les cellules au moyen d'un courant électrique, **caractérisée en ce qu'**elle présente, lors d'une modification de la valeur du pH 7 jusqu'à une valeur pH 8 et à une température de 25 °C, une capacité ou pouvoir tampon d'au moins 20 mmoles *I⁻¹ *pH⁻¹ ainsi qu'une force ionique d'au moins 200 mmoles *I^{-1,}, et **en ce que** la concentration en ions potassium (K⁺) est comprise entre 2 et 6 mmoles * I⁻¹ et la concentration en ions sodium (Na⁺) est comprise entre 100 et 150 mmoles * I⁻¹

2. Solution tampon selon la revendication 1, **caractérisée en ce que** celle-ci présente un pouvoir tampon (pH 7-8, 25 °C) compris entre 22 et 80 mmoles *I⁻¹ *pH⁻¹.

3. Solution tampon selon la revendication 1 ou 2, **caractérisée en ce que** celle-ci présente un pouvoir tampon (pH 7-8, 25 °C) compris entre 40 et 70 mmoles* I⁻¹ *pH⁻¹.

4. Solution tampon selon l'une des revendications 1 à 3, **caractérisée en ce que** celle-ci présente une force ionique comprise entre 200 et 500 mmoles *I⁻¹.

5. Solution tampon selon l'une des revendications 1 à 4, **caractérisée en ce que** celle-ci présente une force ionique comprise entre 250 et 400 mmoles *I⁻¹.

6. Solution tampon selon l'une des revendications 1 à 5, **caractérisée en ce que** celle-ci contient au moins 10 mmoles* I⁻¹ d'ions magnésium (Mg²⁺).

7. Solution tampon selon la revendication 6, **caractérisée en ce que** celle-ci contient 15 à 20 mmoles *I⁻¹ d'ions magnésium.

8. Solution tampon selon l'une des revendications 1 à 7, **caractérisée en ce que** celle-ci contient du chlorure de magnésium (MgCl₂) et/ou du sulfate de magnésium (MgSO₄).

9. Solution tampon selon l'une des revendications 1 à 8, **caractérisée en ce qu'**en tant que substance tampon est contenu le HEPES et/ou le mélange Na₂HPO₄/NaH₂PO₄ et/ou le Tris /HCl et/ou le mélange K₂HPO₄/KH₂PO₄.

10. Solution tampon selon l'une des revendications 1 à 9, **caractérisée en ce que** des composants additionnels, tels que par exemple le chlorure de sodium, le succinate de sodium, le mannitol , le glucose, le lactobionate de sodium et/ou des peptides sont contenus dans la solution tampon.

11. Solution tampon selon l'une des revendications 1 à 10, **caractérisée en ce que** celle-ci contient 4-6 mmoles de KCl, 10-20 mmoles de MgCl₂ et 120-160 mmoles du mélange NA₂HPO₄/NaH₂PO₄ (pH 7,2).

12. Solution tampon selon l'une des revendications 1 à 10, **caractérisée en ce que** celle-ci contient 4-6 mmoles de KCl, 10-20 mmole de MgCl₂, 5-25 mmoles de HEPES et 120-160 mmoles de NA₂HPO₄/NaH₂PO₄ (pH 7,2).

13. Solution tampon selon l'une des revendications 1 à 10, **caractérisée en ce que** celle-ci contient 4 - 6 mmoles de KCl, 10 - 20 mmoles de MgCl₂, 50 - 160 mmoles de NA₂HPO₄/NaH₂PO₄ (pH 7,2) et 5 - 100 mmoles de lactobionate de sodium ou 5 - 100 mmoles de mannitol ou 5 - 100 mmoles de succinate de sodium ou 5 - 100 mmoles de chlorure de sodium.

14. Solution tampon selon l'une des revendications 1 à 10, **caractérisée en ce que** celle-ci contient 4 - 6 mmole de KCl, 10 - 20 mmoles de MgCl₂, 5 - 25 mmoles d'HEPES, 50 - 160 mmoles du mélange NA₂HPO₄/NaH₂PO₄ (pH 7,2) et 5 - 100 mmoles de lactobionate de sodium ou 5 - 100 mmoles de mannitol ou 5 - 100 mmoles de succinate de sodium ou 5 - 100 mmoles de chlorure de sodium.

15. Solution tampon selon l'une des revendications 1 à 10, **caractérisée en ce que** celle-ci contient 4 - 6 mmoles de KCl, 10 - 20 mmoles de MgCl₂ 80 - 100 mmoles de NaCl, 8 - 12 mmoles glucose, 0,3 - 0,5 mmoles de Ca (NO₃)₂, 20 - 25 mmoles HEPES et 50 - 100 mmoles de Tris/HCl ou 30 - 50 mmoles de NA₂HPO₄/NaH₂PO₄ (pH 7,2).

16. Solution tampon selon l'une des revendications 1 à 10, **caractérisée en ce que** celle-ci contient 0,1 - 3,0 mmoles de MgCl₂, 50 - 200 mmoles de K₂HPO₄/KH₂PO₄ (pH 6,5) et/ou 1 - 50 mmoles de mannitol et/ou 1 - 50 mmoles de succinate de sodium.

17. Procédé pour l'introduction de molécules biologiquement actives dans des cellules animales ou humaines au moyen d'un courant électrique, comprenant:
i. la mise en suspension des cellules et la dissolution des molécules biologiquement actives dans une solution tampon, qui présente, lors d'une modification de la valeur du pH depuis pH 7 jusqu'à une valeur pH 8 et à une température de 25 °C, une capacité ou pouvoir tampon d'au moins 20 mmoles * I⁻¹ *pH⁻¹ ainsi une force ionique d'au moins de 200
ii. l'application d'une tension électrique à la suspension.

18. Procédé selon la revendication 17, **caractérisé en ce que** la solution tampon présente un pouvoir tampon (pH 7-8, 25 °C) compris entre 22 et 80 mmoles * I⁻¹ *pH⁻¹.

19. Procédé selon la revendication 17 ou 18, **caractérisé en ce que** la solution tampon présente un pouvoir tampon (pH 7 - 8, 25 °C) compris entre 40 et 70 mmoles * I⁻¹ *pH⁻¹.

20. Procédé selon l'une des revendications 17 à 19, **caractérisé en ce que** la solution tampon présente une force ionique comprise entre 200 et 500 mmoles *I⁻¹.

21. Procédé selon l'une des revendications 17 à 20, **caractérisé en ce que** la solution tampon présente une force ionique comprise entre 250 et 400 mmoles * I⁻¹.

22. Procédé selon l'une des revendications 17 à 21, **caractérisé en ce que** la solution tampon contient au moins 10 mmoles * I⁻¹ d'ions magnésium (Mg²⁺).

23. Procédé selon la revendication 22, **caractérisé en ce que** la solution tampon contient de 15 à 20 mmoles * I⁻¹ d'ions magnésium

24. Procédé selon l'une des revendications 17 à 23, **caractérisé en ce que** la solution tampon contient du chlorure de magnésium (MgCl₂) et/ou du sulfate de magnésium (MgSO₄).

25. Procédé selon l'une des revendications 17 à 24, **caractérisé en ce que** la solution tampon présente, en comparaison du cytoplasme des cellules, une concentration plus faible en ions potassium (K⁺), de préférence de 2 à 6 mmoles * I⁻¹ de K⁺, ainsi qu'une concentration plus élevée en ions sodium (Na⁺), de préférence de 100 à 150 mmoles * I¹⁻ de Na⁺.

26. Procédé selon l'une des revendications 17 à 25, **caractérisé en ce qu'**en tant que substance tampon est contenu le HEPES et/ou le mélange Na₂HPO₄/NaH₂PO₄ et/ou le Tris/HCl et/ou le mélange K₂HPO₄/KH₂PO₄.

27. Procédé selon l'une des revendications 17 à 26, **caractérisé en ce** des composants additionnels, tels que par exemple le chlorure de sodium, le succinate de sodium, le mannitol, le glucose, le lactobionate de sodium et/ou des peptides sont contenus dans la solution tampon.

28. Procédé selon l'une des revendications 17 à 27, **caractérisé en ce que** la solution tampon contient 4-6 mmoles de KCl, 10-20 mmoles de MgCl₂ et 120-160 mmoles du mélange NA₂HPO₄/NaH₂PO₄ (pH 7,2).

29. Procédé selon l'une des revendications 17 à 27, **caractérisé en ce que** la solution tampon contient 4-6 mmoles de KCl, 10-20 mmoles de MgCl₂, 5-25 mmoles d'HEPES et 120-160 mmoles du mélange Na₂HPO₄/NaH₂PO₄ (pH 7,2).

30. Procédé selon l'une des revendications 17 à 27 **caractérisé en ce que** la solution tampon contient 4 - 6 mmoles de KCl, 10 - 20 mmoles de MgCl₂ 50 - 160 mmoles NA₂HPO₄/NaH₂PO₄ (pH 7,2) et 5 - 100 mmoles de lactobionate de sodium ou 5 - 100 mmoles de mannitol ou 5 - 100 mmoles de succinate de sodium ou 5 - 100 mmoles de chlorure de sodium.

31. Procédé selon l'une des revendications 17 à 27, **caractérisé en ce que** la solution tampon contient 4 - 6 mmoles de KCl, 10 - 20 mmoles de MgCl₂ 5 - 25 mmoles d'HEPES, 50 - 160 mmoles du mélange NA₂HPO₄/NaH₂PO₄ (pH 7,2) et 5 - 100 mmoles de lactobionate de sodium ou 5 - 100 mmoles de mannitol ou 5 - 100 mmoles de succinate de sodium ou 5 -100 mmoles de chlorure de sodium.

32. Procédé selon l'une des revendications 17 à 27, **caractérisé en ce que** la solution tampon contient 4 - 6 mmoles de KCl, 10 - 20 mmoles de MgCl₂ 80 - 100 mmoles de NaCl, 8 - 12 mmoles de glucose, 0,3 - 0,5 mmoles de Ca (NO₃)₂, 20 - 25 mmoles d'HEPES et 50 - 100 mmoles de Tris/HCl ou 30 - 50 mmoles de Na₂HPO₄/NaH₂PO₄(pH 7,2).

33. Procédé selon l'une des revendications 17 à 27, **caractérisé en ce que** la solution tampon contient 0,1 - 3,0 mmoles de MgCl₂, 50 - 200 mmoles de K₂HPO₄/PO₄ (pH 6,5) et/ou 1 - 50 mmoles de mannitol et/ou 1 - 50 mmoles de succinate de sodium.

34. Procédé selon l'une des revendications 17 à 33, **caractérisé en ce que** l'introduction des molécules biologiquement actives dans les cellules est réalisée via une impulsion de courant avec une intensité de champ de 2 à 10 kV *cm⁻¹ et une durée de 10 à 200 µs ainsi qu'une densité de courant d'au moins 2 A*cm⁻².

35. Procédé selon la revendication 34, **caractérisé en ce que** l'introduction des molécules biologiquement actives dans les cellules est réalisée via un flux électrique succédant sans interruption à l'impulsion de courant, avec une densité de courant de 2 A*cm⁻² à 14 A*cm⁻², de préférence jusqu'à 5 A*cm⁻² et une durée de 1 à 100 ms, de préférence jusqu'à 50 ms.

36. Procédé selon la revendication 34 ou 35, **caractérisé en ce que** les molécules biologiquement actives sont transférées au moyen du courant électrique dans le noyau cellulaire des cellules animales ou humaines.

37. Procédé selon l'une des revendications 17 à 36, **caractérisé en ce que** des acides nucléiques, des protéines, des peptides ou d'autres molécules biologiquement actives sont introduits dans des cellules animales ou humaines, au repos ou partiellement actives.

38. Procédé selon l'une des revendications 17 à 37, **caractérisé en ce que** des acides nucléiques, des protéines ou des peptides sont introduits dans des cellules primaires, animales ou humaines.

39. Procédé selon la revendication 37 ou 38, **caractérisé en ce que** les acides nucléiques sont présents sous forme de complexe ou en combinaison avec des peptides, des protéines ou d'autres molécules biologiquement actives.

40. Procédé selon l'une des revendications 17 à 39, **caractérisé en ce que** les cellules sont des cellules primaires de sang humain, des cellules, à potentiel multiple, précurseurs du sang humain, des fibroblastes humains, des cellules humaines avec endothélium, des cellules musculaires ou des mélanocytes.
